# EUROPEAN PATENT SPECIFICATION

(11) **EP 4 410 365 B1**
(45) Date of publication and mention of the grant of the patent: **04.11.2026**
(21) Application number: 23154117.8
(22) Date of filing: 31.01.2023
(51) Int. Cl.: A61N 5/10

(54) **METHOD FOR DEFINING A SCANNING SEQUENCE FOR RADIATION TREATMENT OF A TARGET VOLUME, BY PENCIL BEAM SCANNING (PBS) AT ULTRA HIGH DOSE DEPOSITION RATE (HDR)**
VERFAHREN ZUR DEFINITION EINER ABTASTSEQUENZ ZUR STRAHLENBEHANDLUNG EINES ZIELVOLUMENS DURCH STIFTSTRAHLABTASTUNG (PBS) MIT ULTRAHOHER DOSISABLAGERUNGSRATE (HDR)
PROCÉDÉ DE DÉFINITION D'UNE SÉQUENCE DE BALAYAGE POUR LE TRAITEMENT PAR RAYONNEMENT D'UN VOLUME CIBLE, PAR BALAYAGE À FAISCEAU CRAYON (PLS) À TRÈS HAUT DÉBIT DE DÉPÔT DE DOSE

(43) Date of publication of application: 07.08.2024
(73) Proprietor: Ion Beam Applications, 1348 Louvain-la-Neuve (BE)
(72) Inventor: LABARBE, Rudi, 1348 Louvain-la-Neuve (BE); HOTOIU, Lucian, 1348 Louvain-la-Neuve (BE); PIN, Arnaud, 1348 Louvain-la-Neuve (BE)
(74) Representative: Gevers Patents

(56) References cited:
- EP-A1- 3 932 482
- US-A1- 2020 298 025
- US-A1- 2023 023 312

## Description

### FIELD OF THE INVENTION

The present invention relates to a method for defining a scanning sequence for treatment by radiation with charged particles beams, preferably with proton beams, and for depositing doses (Dj) applied by pencil beam scanning (PBS) in an energy monolayer into a target volume (Vt) comprising tumoral cells enclosed within a peripheral surface. The target volume (Vt) comprises healthy cells confined in a critical volume (Vc), which have to be spared by the treatment. The sequence must ensure that the doses (Dj) are deposited at an ultra-high dose deposition rate (HDR) in a significant part of the critical volume (Vc) comprising the healthy cells which must be spared.

The present method ensures that all doses (Dj) deposited onto a given specific volume (vi) by, on the one hand, beamlets directly oriented towards the given specific volume (vi) and, on the other hand, also by beamlets directed to neighbouring specific volumes but whose deposited dose impinges on the given specific volume (vi), are deposited in combination within a cumulative dose deposition time, ΔTi, short enough to guarantee that the cumulative dose, Σ_{{tⱼ}} Dj, was deposited at HDR onto the critical volume. The method is adapted for treating target volumes having complex geometries. The sum of specific volume (vi) defines the critical volume (Vc), i.e. the volume where doses are to be deposited at an ultra-high dose deposition rate.

### BACKGROUND OF THE INVENTION

Radiation therapy with particles or waves, such as electron beams, protons beams, heavy ions beams, x-rays, *γ*-rays, and the like, has become an essential tool for treating patients with tumours. Pencil beam scanning (PBS) is a technique consisting of steering a beam of charged particles towards a target volume comprising tumoral cells. PBS reduces unnecessary radiation exposure to surrounding non-cancerous cells by shaping the area being treated to mirror the tumour geometry. Beside the geometry of the target, PBS allows local tuning of the intensity of a beamlets depending on the position of the irradiated cells within the target. Pencil beam scanning can treat a tumour with a single beam composed of various beamlets or with multiple beams of different orientations each composed of various beamlets, sometimes called intensity modulated proton therapy (IMPT). Since both tumoural cells and healthy cells are damaged by such radiations, a major challenge in cancer treatment is to ensure that the tumoural cells are effectively destroyed or killed, while as many healthy cells as possible are spared, in particular those adjacent to the tumoural cells.

X-rays deposit most of their energy near the level of the skin, and the deposited energy decreases with depth. Healthy tissues located upstream of a target volume of tumoural cells therefore receive a higher dose than the tumoural cells of target volume. By contrast, charged particle beams, in particular protons deposit most of their energy close to the end of their beam path, forming a so-called Bragg peak. By superposing a number of beamlets with their respective Bragg peaks staggered in depth, a sum of individual Bragg Peaks (SOBP) can be defined spanning a whole depth of a specific volume. The healthy cells located upstream of the volume of tumoural cells crossed by a proton beam therefore receive a lower dose than the tumoural cells in the specific volume. Consequently, proton therapy is well suited for depositing high doses in deep seated tumours.

Historically, treatment by radiation therapy included the delivery of radiation doses to the treated cells at a conventional dose deposition rate (CDR) lower than 1 Gy / s. With rare exceptions, current radiation therapy facilities deliver dose-rates around 0.03 Gy / s and most clinical protocols involve daily delivery of N target fraction doses of 2 to 15 Gy cumulated to reach the total target dose) which may exceed the tolerance limit of normal tissues located in the radiation field, thus damaging them together with the tumoural cells. Recently, it has been observed that a same dose had different effects on healthy cells but not on tumoral cells when deposited at conventional dose deposition rates (CDR) or at ultra-high dose deposition rate (HDR). HDR can be one or more orders of magnitude larger than conventional dose deposition rates (CDR) usually applied. Deposition of a dose at ultra-high dose deposition rates (HDR) is also referred to as FLASH-radiotherapy (= FLASH-RT). It has been demonstrated experimentally on animals and on various organs, that dose deposition at HDR can significantly spare healthy tissues in comparison with conventional deposition of a same dose at CDR and, at the same time, tumoural cells respond same or even possibly better to HDR deposition than to CDR deposition. For example, FLASH-RT reportedly elicits in mice a dramatic decrease of the incidence of lung fibrosis, of memory loss subsequent to brain irradiation, and of necrosis of the small intestine whilst keeping the anti-tumour efficiency unchanged. Such specific normal tissue sparing has been confirmed in large animals and a patient with cutaneous lymphoma has already been treated with FLASH-RT. Many treatment centres, however, do not dispose of equipment capable of delivering dose at HDR in a time of the order of the ms or s or dispose of equipment that can be modified to deliver HDR only in a very limited field size. Such effects were observed for dose deposition rates of the order of 1 Gy / s and higher.

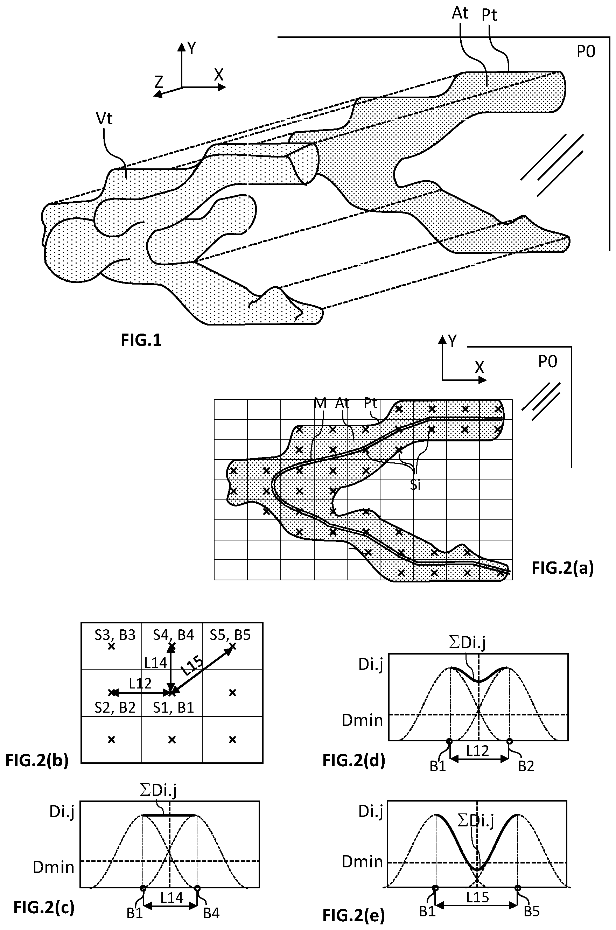

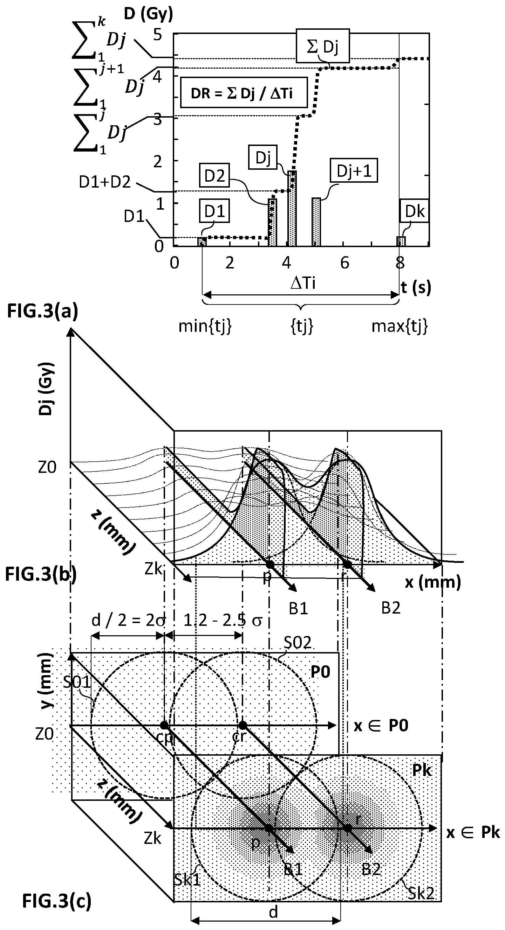

As illustrated in Figures 3(b) and 3(c), a single beamlet propagating along a central irradiation axis (Z) radially deposits doses approximately according to a Gaussian distribution curve, normal to the central irradiation axis (Z). In Figure 3(b), Gaussian deposition curves are illustrated along a direction (x) normal to the central irradiation axis (Z). Figure 3(c) illustrates with shade codes the dose distribution deposited radially by a beamlet. As can be seen in Figures 3(b) and 3(c), the beam axes of two adjacent proton beamlets in PBS are distributed according to a spot positions pattern (x, y) and are generally separated from one another by a distance comprised between 1.2 and 2.5 times *σ*, preferably 1.3 to 1.5 times *σ*, wherein *σ*² is the variance of the Gaussian dose distribution of a beamlet on the surface plane (Pk) normal to the central irradiation axis (Z). It follows that a beamlet (B1) deposits a dose onto an area of the plane (Pk) which impinges over the zone of dose deposition by a second beamlet (B2) adjacent to the first beamlet (B1), yielding the wavy distribution curve illustrated in Figure 3(b) (two lumps only are illustrated, but this shape becomes more complex with additional adjacent beamlets propagating along the central irradiation axis (Z) at a different y-positions from the first and second beamlets (B1, B2). Such overlap is essential to ensure a substantially uniform dose deposition onto a whole area on the plane (Pk). This has, however, an unexpected effect on the FLASH effect, illustrated in Figure 3(a). The amplitude of the wavy distribution curve depends *inter alia* on the distance between two adjacent spots and on the number of spots delivering a dose at a given location (cf. also Figures 2(b) to 2(e)).

Figure 3(a) illustrates an example of doses deposited as a function of time in a specific volume (vi). It can be seen that at a time (t) of approximately 4s a dose (Dj) was deposited by a beamlet (Bj) centred on the specific volume (vi), at ultra-high dose rate (HDR) well within FLASH-RT effect. The same specific volume (vi), however received doses (D1, D2, Dj+1, Dk) which "leaked" from adjacent beamlets (B1, B2, Bj+1, Bk)) aimed at neighbouring specific volumes at times comprised between 1 and 8 s, also each deposited at ultra-high dose deposition rates. The total cumulated dose Σ_{{tⱼ}} Dj) deposited in the specific volume (vi) is about 4.5 Gy (i.e., Σ_{{tⱼ}} Dj = 4.5 Gy). Although the doses of each beamlet (B1 to Bk) were deposited at an ultra-high dose deposition rate greater than 1 Gy / s, the overall irradiation time (ΔTi) of dose deposition onto the specific volume (vi) in the example of Figure 3(a) is such that the overall dose deposition rate onto the specific volume (vi), DR(vi) = Σ_{{tⱼ}} Dj/ΔTi = 4.5 Gy / 7 s = 0.64 Gy / s, is lower than 1 Gy / s, thus losing the FLASH-RT effect. This example illustrates the danger of losing the FLASH-RT effect in spite of having deposited doses at HDR in a whole region, because it is not the instantaneous dose deposition rate alone that counts, but the cumulated dose rate (Σ_{{tⱼ}} Dj/ΔTi) instead. Since all individual doses were deposited at ultra-high dose deposition rates (HDR), the clinician may be of the false impression that FLASH-RT effect was present thus sparing the healthy tissues. This impression, however, may be wrong in case the total dose was deposited over a cumulated period of time (ΔTi) such that at the overall deposition rate DR(vi) < 1 Gy / s, the cumulated dose was deposited at CDR instead of HDR, thus exceeding the dose admitted at CDR for the healthy cells. In the present example of Figure 3(a), depositing a cumulated dose, Σ_{{tⱼ}} Dj = 4.5 Gy with five beamlets (B1 to Bk), each at HDR and distributed over a cumulated time ΔTi = 7 s is too long to obtain the FLASH-RT effect.

EP3932482 identifies the problem of losing the FLASH-RT effect in case a specific volume receives doses from beamlets aimed at neighbouring specific volumes at time intervals which are too longs. To solve that problem, EP3932482 describes a treatment plan system comprising a beamlets scanning sequence stage configured for defining a scanning sequence of irradiation of the beamlets onto an array of specific volumes by optimising a time sequence of beamlets emission such that at the end of an irradiation operation, a dose is deposited onto at least 50% of each specific volume at a mean deposition rate greater than 1 Gy / s. Various embodiments of the beamlets scanning sequence stage are described. For example, a scarf sequence unit cell is defined by first defining an initial spot to be irradiated first by a first beamlet. Successive second to n^{th} spots are defined, each sequentially adjacent to one another and all aligned along a first direction, for successively receiving 2^{nd} to n^{th} beamlets. At the n^{th} spot, the sequence includes a next (n+1)^{th} spot adjacent thereto along a second direction, preferably perpendicular to the first direction, prior to further extending along the first direction again to cover (n+2)^{th} to 2n^{th} spots, wherein the 2n^{th} spot is adjacent to the first spot. At the 2n^{th} spot, the sequence extends one step along the second direction, opposite to the first spot, to cover a (2n+1)^{th} spot and so on, thus defining a winding scarf extending along the second direction and spanning along the first direction over a width of the distance separating the first spot from the n^{th} spot.

The scarf sequence unit cell defined in EP3932482 is very efficient for defining an irradiation sequence ensuring that a sum of a percentile of all the doses deposited by one or more beamlets onto a specific volume are deposited at a ultra-high dose deposition rate (HDR), wherein the percentile is at least 95%. The problem is that it applies to tumours having relatively simple and substantially linear geometries and is ill-fitted for defining an irradiation sequence having a complex geometry as e.g., illustrated in Figure 1. Further, US 2023/023312 discloses a method for defining a scanning sequence in a FLASH proton radiotherapy, using pencil beam scanning. Irradiation spots are defined and a continuous scanning sequence in a plane is established

There therefore remains a need for a method for defining an irradiation sequence of a target volume comprising tumoural cells ensuring that the FLASH-RT effect is obtained in the required percentile. The present invention solves the problem of ensuring that a target treated by PBS of charged particles is effectively irradiated at HDR where required taking account of any overlapping dose deposition distribution of all beamlets leaking over a given specific volume to be treated. These and other advantages are described in more detail in continuation. These and other advantages of the present invention are explained more in detail in the following sections.

### SUMMARY OF THE INVENTION

The present invention concerns a method for defining a scanning sequence for treatment by radiation with charged particles beams, preferably with proton beams, wherein the method ensures that doses (Dj) are deposited by pencil beam scanning (PBS) in an energy monolayer onto a target volume (Vt) comprising tumoral cells enclosed within a peripheral surface and at a ultra-high dose deposition rate (HDR) onto a significant fraction of specific volumes (vi) defining a critical volume (Vc) comprising healthy cells, wherein HDR is defined as a dose deposition rate, HDR = Σ_{{tⱼ}} Dj/ΔTi ≥ 1 Gy / s, wherein Di is a dose deposited onto a specific volume (vi) of the critical volume (Vc) by a j^{th} pencil beam at time tj after beginning of the irradiation, and wherein ΔTi = max({tj}) - min({tj}), is a time spanning between an initial dose deposition time (min({tj})) when a first dose ≥ Dmin is deposited into the specific volume and a final dose deposition time (max({tj})) when a last dose ≥ Dmin is deposited into the specific volume, wherein Dmin is a lower dose boundary. The method comprises,
- defining a target area (At) bounded by a target perimeter (Pt) by projection of the target volume (Vt) including the critical volume (Vc) onto a surface plane (P0 = (Y, Z)) normal to a central irradiation axis (X),
- defining a spot diameter and spot position pattern on the surface plane (P0) of spots (Sj) distributed at least within the target perimeter (Pt) such as to cover at least a whole area defined within the target perimeter (Pt),
- establishing a scarf scanning sequence defining a continuous path connecting all the spots (Sj) to one another and configured for minimizing the cumulative dose deposition time (Δ*Tᵢ*) in all the specific volumes (vi), such as to ensure HDR-deposition into all specific volume (vi).

The scarf scanning sequence is established as follows,
- for a given value of the proton beam current, defining a HDR-scanning distance (L) a particle beam can scan in a two-way return path while ensuring that the dose (Dj) is deposited at HDR deposition rate into all specific volumes (vi) touched by the particle beam along the two-way return path thereof,
- defining a pseudo-mediatrix (M) characterizing a geometry of the target perimeter (Pt),
- the continuous path comprises a series of path sections which connect a number of adjacent spots distributed on one side or on either side of the pseudo-mediatrix wherein,
   ∘ each path section intersects or crosses once the pseudo-mediatrix (M) at different points distributed along a length of the pseudo-mediatrix,
   ∘ the path sections are connected to one another two by two forming a series of return path sections of length smaller than or equal to the HDR-scanning distance (L).

The return path sections can be formed by connecting an end spot of a first path-section defined as a last spot connected in the first path section to a second path section adjacent to the first path section by connecting the end spot of the first section to a spot of the second path-section located either,
- at a shortest distance from and on the same side of the pseudo-mediatrix as the last spot, or
- at a longest distance from and on the other side of the pseudo-mediatrix (M) as the last spot.

There are different ways to define the pseudo-mediatrix (M). In a first embodiment, the pseudo-mediatrix (M) can be defined as follows,
- define a set of reference points (RP) distributed over a whole of the target area (At); wherein the reference points (RP) preferably are centres of spots (Sj),
- select a first reference point (RP),
- define a radius of influence (R),
- define a set of influence points defined as the reference points (RP) enclosed within a circle of influence of radius of influence (R) centred on the first reference point (RP)
- compute an ellipse of influence having a same second moment of inertia as the set of all influence points in the circle of influence,
- identify the major axis of the ellipse of influence,
- define a flow vector starting from the first reference point (RP), of direction parallel to the major axis, and of arbitrary length,
- repeat the foregoing steps for all reference points (RP) with circles of influence of the same radius of influence (R),
- connect the flow vectors to one another to define flow lines,
- select one flow line extending from one end to another end of the target perimeter (Pt) as forming the pseudo-mediatrix (M).

In a second embodiment, the pseudo-mediatrix (M) can be defined as follows,
- defining a first connecting segment of first direction intersecting the target perimeter at two intersecting points, wherein a segment length is defined as a distance separating the two intersecting points,
- defining a first mid-point (MP) of the first connecting segment, defined as the point located at a centre of the connecting segment,
- repeating the foregoing steps with further connecting segments parallel to the first direction,
- connecting all mid-points (MP) to define the pseudo-mediatrix (M).

It is preferred that the first connecting segment and further connecting segments each passes through the centre of at least one spot (Sj), preferably of at least two spots (Sj).

In a third embodiment, the pseudo mediatrix (M) is defined as follows,
- define a reference rectangle of given dimensions and orientation,
- pave the target area (At) with a series of reference rectangles such as to cover a whole of the target area,
- for a first reference rectangle, identify the spots (Sj) enclosed therein,
- determine the geometric median (GM) of the spots (Sj) enclosed in the first reference rectangle, wherein the geometric median forms a first dot of the pseudo-mediatrix (M),
- repeat the last two steps for all the reference rectangles to form a string of dots,
- connecting the dots thus formed to form the pseudo-mediatrix (M).

The scarf scanning sequence can be established as follows,
- define a rectangular reference box (RB) with major edges of length (Lb) and with minor edges of width (Wb < Lb) having a major median extending parallel to the major edges, wherein Lb < L and Wb is smaller than twice a shortest distance (ds) separating the centres of two adjacent spots (Sj) (i.e., Wb < 2 ds),
- pave the target area (At) with a series of reference boxes (RB), with the major median crossing the pseudo-mediatrix (M) and forming therewith a given angle, preferably of 90°, such as to enclose all the spots (Sj) in at least one reference box,
- defining a path section with spots (Sj) enclosed in one reference box, each spot (Sj) belonging to a single path section even if enclosed in more than one reference box,
- connecting the path sections to one another two by two to form the continuous path.

The lower dose boundary (Dmin) can be defined either
- as an absolute dose value, below which a dose is considered to have an insignificant effect, or
- as a percentage of the total dose deposited to the specific volume (vi), preferably Dmin is at least 5% of Σ_{{tⱼ}} Dj deposited onto the specific volume (vi), more preferably at least 10% of Σ_{{tⱼ}} Dj.

### BRIEF DESCRIPTION OF THE FIGURES

On these figures,
**Fig.** 1 shows an example of a target volume (Vt) comprising tumoural cells, and the projection thereof onto a surface plane (P0 = (Y, Z)) normal to a central irradiation axis (X), defining a target area (At) bounded by a target perimeter (Pt).
**Fig.** 2(a) illustrates the target area (At) of Figure 1 paved by an array of spots (Si) corresponding to a central point of the projections on the surface plane (P9) of the individual specific volumes which the doses are to be deposited onto. The double-line represents a pseudo-mediatrix (M).
**Fig. 2(b)** shows the distances separating two adjacent beams (B1-B5) along different directions.
**Fig. 2(c) to 2(e)** show the doses deposited onto two adjacent specific volumes successively irradiated by two beamlets centred on each of the specific volumes separated from one another by a distance, L12, L14, and L15, respectively, as defined in Figure 2(b).
**Fig. 3(a)** shows the cumulative dose deposited onto a specific volume by successive beamlets aligned on the specific volume and on neighbouring specific volumes, separated from one another by a time sequence such that the FLASH-RT effect is not obtained.
**Fig. 3(b)** shows the doses deposited over a plane (x, z) by two proton beamlets (B1, B2), with overlapping gaussian dose distribution curves along the X-axis,
**Fig. 3(c)** shows a 3D view (x, y, z) of the approximately cylindrical propagation of the two beamlets (B1, B2) along the central irradiation axis (Z), the shades of grey illustrating the doses deposited on a plane (Pk) located at a depth (Zk) from the surface of the skin of a patient.
**Fig. 4.1 to 4.7** show the cumulated dose deposited onto each of seven specific volumes centred on a series of spots S1 to S7 according to a first irradiation sequence or beam trajectory.
**Fig. 5.1 to 5.7** show the cumulated dose deposited onto each of the same seven specific volumes as in Figures 4.1 to 4.7, but according to a second irradiation sequence or irradiation path (IP)different from the first irradiation sequence.
**Fig. 6(a) & 6(b)** show an embodiment of construction of the pseudo-mediatrix according to the best-fit ellipse method based on reference points.
**Fig. 7(a) & 7(b)** show the embodiment of construction of the pseudo-mediatrix according to the best-fit ellipse method based on reference points wherein the reference points are spots.
**Fig.8** shows an embodiment of construction of the pseudo-mediatrix according to the mid-point method.
**Fig.9** shows an embodiment of construction of the pseudo-mediatrix according to the geometric median method.
**Fig. 10(a) to 10(c)** show three different embodiments for paving the target area with reference boxes. Figure 10(a) has a pseudo-mediatrix (M) defined by the best-fit ellipse method as illustrated in Figures 7(a)&7(b), Figure 10(b) comprises a hexagonal pattern of spots and a pseudo-mediatrix (M) defined by the mid-point method as illustrated in Figure 8, and Figure 10(c) has a pseudo-mediatrix (M) defined by the geometric median method as illustrated in Figure 9.
**Fig. 11(a) to 11(c)** show three embodiments of how to join two-by-two the path segments enclosed in the reference boxes of Figures 10(a) to 10(c), respectively.

### DETAILED DESCRIPTION.

The present invention concerns a method for defining a scanning sequence for treatment by radiation with charged particles beams, preferably with proton beams. Such radiation treatments kill tumoural cells enclosed within a target volume (Vt). The target volume (Vt), however, is surrounded by healthy cells and may contain healthy cells within the target volume (Vt) which are exposed to the same level of dose deposition as the tumoural cells. It is a challenge to avoid collateral damages when killing the tumoural cells by sparing the healthy cells. Maximum total dose acceptable to the healthy cells can be defined relative to a Normal Tissue Complication Probability (NTCP) which defines the probability of a given tissue of developing complications upon exposure to a given radiation. Values of boundary doses of radiation yielding a given value of the NTCP for a selection of organs are available in the literature. The invention is defined in the following claims.

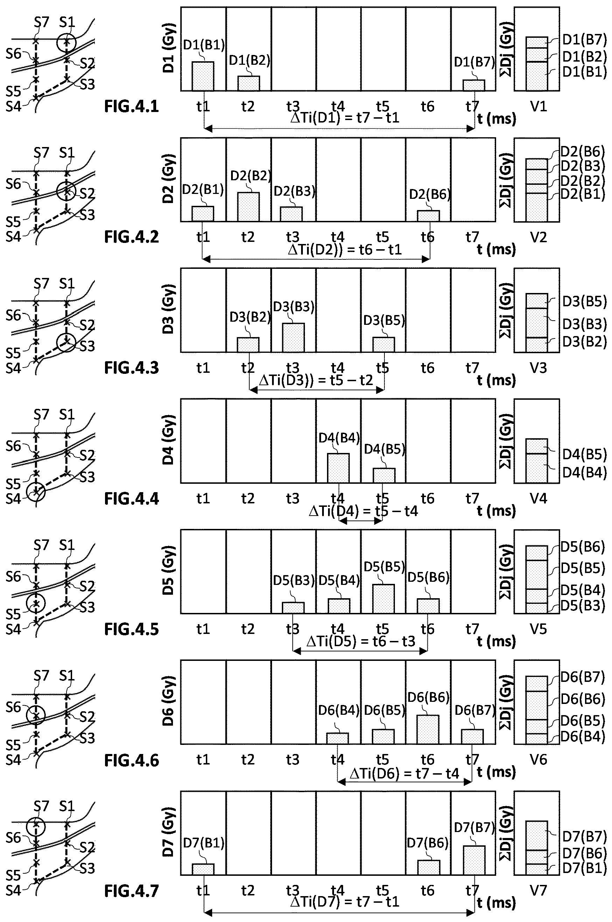

FLASH-RT, wherein doses are deposited at ultra-high deposition rates, could possibly be part of a solution to solve this difficult problem. Tumoural cells seem to be equally killed by radiations regardless of the dose deposition rate, whilst healthy cells can receive higher doses at ultra-high deposition rates for a given value of NTCP, than at conventional deposition rates (cf. e.g., EP3932481, Tables 1 and 2, and Figures 4(a) and 4(b)). Because a given specific volumes treated by PBS receives doses from beamlets (Bj) centred on the given specific volume but also from beamlets centred on adjacent specific volumes, it may not be possible to deposit the desired doses (Σ_{{tⱼ}} Dj) at HDR on all specific volumes if the scanning sequence includes scanning neighbouring spots over a time span (ΔTi) too high to yield ultra-high dose deposition rate in all spots. The desired dose (Σ_{{tj}} Dj) is deposited into the specific volume if the dose deposition rate, HDR = Σ_{{tⱼ}} Dj/ΔTi ≥ 1 Gy / s, wherein Dj is a dose deposited onto a specific volume (vi) of the critical volume (Vc) of the target volume (Vt) by a j^{th} pencil beam at time tj after beginning of the irradiation, and wherein ΔTi = max({tj}) - min({tj}), is a time spanning between an initial dose deposition time (min({tj})) when a first dose is deposited into the specific volume and a final dose deposition time (max({tj})) when a last dose is deposited into the specific volume, summing up the desired dose (Σ_{{tj}} Dj). In other words, ΔTi = max({tj}) - min({tj}), is a cumulative dose deposition time during which beamlets have deposited onto the specific volume (vi) a significant dose contribution, Dj ≥ Dₘᵢₙ onto the specific volume (vi), wherein Dmin is a lower dose boundary

The target volume (Vt) must comprise the tumoural cells to be killed but also any healthy cells affected by the radiation treatment of the tumoural cells. These healthy cells can surround a group of the tumoural cells. They can be mixed with the tumoural cells, or they can be located upstream of the tumoural cells, such as to be traversed by the beamlets before they reach the tumoural cells. The healthy cells are enclosed in a critical volume (Vc) which needs not necessarily be continuous and can be distributed in several subvolumes. In the following, the treatment of a continuous critical volume or of a single subvolume is described, as the same method can be applied to each subvolume if required. The method of the present invention allows ensuring that doses (Dj) are deposited by pencil beam scanning (PBS) in an energy monolayer at a ultra-high dose deposition rate (HDR) onto a significant fraction of specific volumes (vi) defining the critical volume (Vc) comprising healthy cells enclosed within a peripheral surface defining the target volume (Vt) containing the tumoral cells. The critical volume (Vc) is fully enclosed within the target volume (Vt). As mentioned supra, HDR is defined as a dose deposition rate, HDR = Σ_{{tⱼ}} Dj/ΔTi ≥ 1 Gy / s, wherein,
- Dj is a dose deposited onto a specific volume (vi) of the critical volume by a j^{th} pencil beam at time ti after beginning of the irradiation,
- Σ_{{tⱼ}} Dj is a sum of a percentile of all the doses deposited by one or more beamlets onto a given volume, wherein the percentile is at least 95%, preferably at least 98%, and
- ΔTi = max({tj}) - min({tj}), is a cumulative dose deposition time during which beamlets have deposited onto the specific volume (vi) a significant dose contribution, Dj ≥ Dₘᵢₙ onto the specific volume (vi), wherein Dmin is a lower dose boundary,

The method comprises defining a target area (At), a spot diameter and position pattern, and establishing a scarf scanning sequence of irradiation of specific volumes (vi) by a sequence of beamlets (Bj).

The target area (At) is bounded by a target perimeter (Pt) by projection of the target volume onto a surface plane (P0 = (Y, Z)) normal to a central irradiation axis (X). An array of spots (Sj) is defined, characterized by the spot diameter and the spot position pattern on the surface plane (P0) of spots. The spots (Sj) of the array are distributed at least within the target perimeter (Pt) such as to cover at least a whole area defined within the target perimeter (Pt).

The scarf scanning sequence is established to define a continuous path connecting all the spots (Sj) to one another. It is configured for minimizing the cumulative dose deposition time (Δ*Tᵢ*) in each specific volumes (vi), such as to ensure HDR-deposition onto all specific volumes (vi). The gist of the present invention is, unlike in EP3932482, to allow the scarf to follow a curved trajectory rather than a straight trajectory, as is explained below.

For a given value of the proton beam current, defining a HDR-scanning distance (L) a particle beam can scan in a two-way return path while ensuring that the dose (Dj) is deposited at HDR deposition rate into all specific volumes (vi) touched by the particle beamlet along the two-way return path thereof. Note that the path followed by the beamlet in the two-way return path has a length of 2L.

A pseudo-mediatrix (M) is defined, which characterizes a geometry of the target perimeter (Pt). Different methods for defining the pseudo-mediatrix (M) are discussed below.

As the pseudo-mediatrix is defined, the continuous path comprises a series of path sections which connect a number of adjacent spots (Sj) distributed on one side or on either side of the pseudo-mediatrix (M). There are enough path sections to ensure that all spots (Sj) are enclosed in at least one path section. Each path section intersects or crosses once the pseudo-mediatrix (M) at different points distributed along a length of the pseudo-mediatrix. The path sections are connected to one another two by two forming a series of return path sections of length smaller than or equal to the HDR-scanning distance (L). The combination of all the return path sections defines the scanning sequence.

### TARGET AREA (At) AND ARRAY OF SPOTS (Sj)

The target area (At) is simply obtained by projecting the target volume on a plane (P0) normal to the central irradiation axis (Z). The beamlets (Bj) exit out of a nozzle which can be considered as forming the apex of a scanning cone whose base encloses at least the target area (At). The central irradiation axis (Z) is defined by the axial axis of the nozzle. The beamlets are able to scan within the scanning cone and thus cover the whole target area (At). If the tumour is too large to allow the beamlets to scan over the whole corresponding target area (At), the irradiation session must be carried out in two or more) stages, including moving the patient to cover the whole target area. Since the distance of the nozzle to the plane (P0) is substantially larger than a diameter of the target area (At), the beamlets can be approximated to be about parallel to the central irradiation axis (Z), albeit this is not strictly true, because of the aperture of the scanning cone.

The intensity of the beamlets defines the number of charged particles discharged per unit time and thus the dose deposition rate at one spot (Sj). The beamlets intensity has an upper limit attached to the type of particle accelerator used. In the present invention, the particle accelerator must be configured for dispensing beamlets of sufficient intensity to deposit a required dose (Dj) onto each individual specific volume (vi) at HDR (i.e., Dj / tj ≥ 1 Gy / s). This is a necessary, non-sufficient condition for ensuring that at the end of an irradiation session, the doses are deposited at HDR in substantially the whole of the target volume (Vt) (i.e., Σ_{{tⱼ}} Dj / Δtj ≥ 1 Gy / s, wherein Δtj = max({tj}) - min({tj})).

As schematically illustrated in Figure 3(b), to paint the target volume (Vt) by PBS in a single layer, several superposed and coaxial beamlets centred on each spot (Sj) are emitted to define a desired shape of the SOBP as a function of the geometry of the target tissue produced by each beamlet, the intensity of the beamlets, and the like. The spot position pattern (x, y) on the surface plane (P0) as well as the diameter of a spot (S01, S02) formed by one or a series of coaxial beamlets must be defined. The skilled person knows how to optimally define the positions and diameters of the spots.

### Diameter (d) of a Spot

As shown in Figure 3(c), a spot (S01, S02) of diameter (d) is formed on the surface plane (P0) (for example at the level of the skin of the patient) by a beamlet (B1, B2) propagating along a beamlet axis substantially parallel to the central irradiation axis (Z) and intersecting substantially perpendicularly the surface plane (P0) at a spot centre (cp, cr), wherein the surface plane (P0) contacts the skin of the patient at least at the spot centre. The diameter of a spot depends on the dose distribution deposited on the surface plane (P0) by said beamlet. The dose distribution on the surface plane follows a substantially normal or Gaussian distribution, as illustrated in Figure 3(b), centred on the intersection point (cp, cr) of the beamlet axis with the surface plane (P0) and of variance σ². The dose (Dj) deposited by a beamlet at any given depth (Zk, with k = 0 - n) is maximal at an intersection point (cp, cr), between the beamlet axis and the corresponding plane (Pk, with k = 0 - n). The spot diameter (d) can be defined as d = 2 x (2 σ) centred on the corresponding spot centre (cp, cr), which comprises 95.4% of the dose distribution or, alternatively, d = 2 x (3σ), which comprises 99.7% of the dose distribution. Preferably, the spot diameter is defined as, d = 2 x (2 σ).

Referring to Figure 3(c), the spot diameter (d) is herein measured at the isocentre plane (Pk), and is preferably a multiple of *σ* (*σ*² = variance), d = m *σ*, preferably with m = 2 , 3 or 4. As shown in Figure 3(b), the value of *σ* of the dose distribution deposited by a beamlet (Bj) at the depth of the target must be considered to compute a spots spacing yielding a uniform dose in the target. t.

### Spot Position Pattern (x, y)

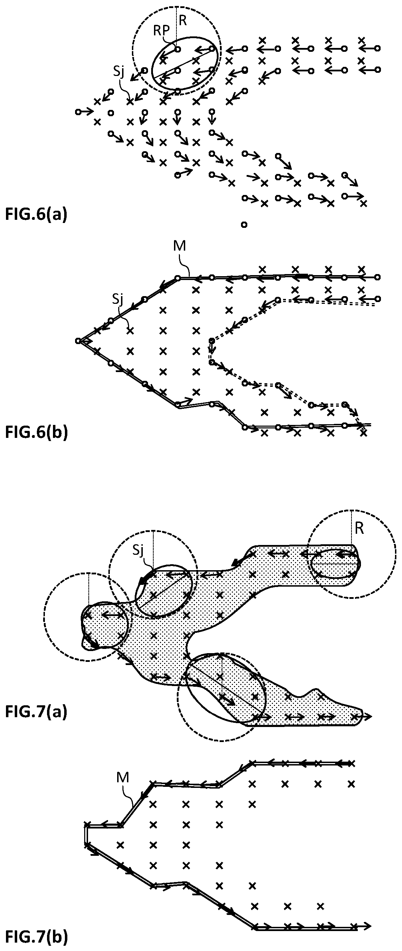

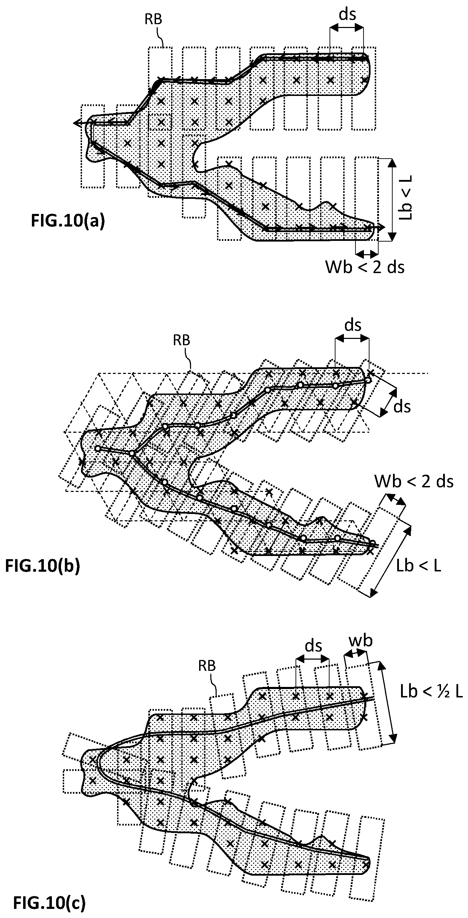

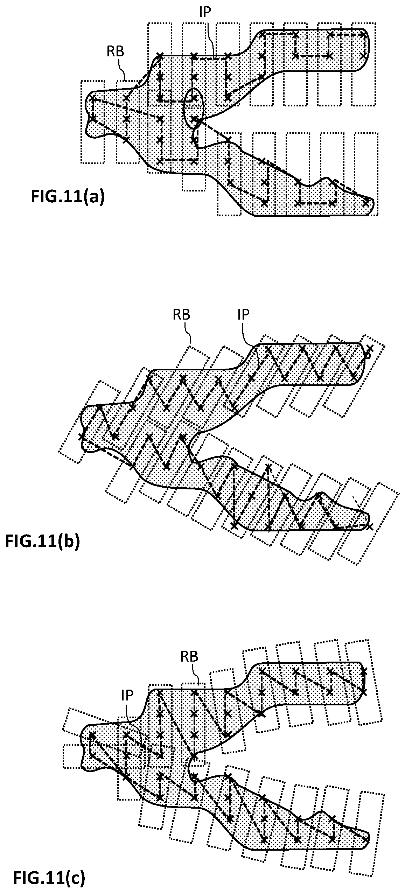

Irradiation by pencil beam scanning (PBS) requires a spot position pattern (x, y) to be defined on the surface plane (P0) of the spots (S01, S02) formed by different beamlets substantially parallel to the central irradiation axis (Z). The spots positions pattern (x, y) must ensure that the spots cover an entirety of the target area (At), such that the required doses can be deposited onto substantially the whole of the target volume (Vt). Knowing the spot diameter (d) at the depth of the target, the spot positions pattern (x, y) on the surface plane (P0) is automatically determined by projection onto the surface plane, parallel to the central irradiation axis (Z) to homogeneously cover an area enclosed within the target perimeter (Pt). An example of rectangular spot position pattern (x, y) is schematically illustrated in Figure 2(a) with the centres of the spots being indicated with crosses. Figures 10(b) and 11(b) show an alternative spot pattern arranged according to a hexagonal distribution. With the exception of Figures 10(b) and 11(b), the same spot position pattern as in Figure 2(a) is reproduced in Figures 6 to 11 for consistency.

A homogeneous dose deposition onto substantially the whole of the target volume (Vt) can be produced by centring the beamlets (Bj) on an array of spots separated from one another by a distance comprised between 1.2 and 2.5 times σ, preferably 1.3 to 1.5 times σ, wherein σ² is the variance of the Gaussian dose distribution formed by a proton beamlet (Bj) at any plane (Pk) normal to the central irradiation axis (Z) at the depth of the target. With such spot position pattern, the impingement of the Gaussian distributions of two adjacent spots levels off to a substantially constant value, as shown in Figure 2(c). The distances separating two adjacent spot axes needs not be constant for all pair of adjacent spots, and they can vary from one another to fit the geometry of the peripheral surface. Regular arrays of spots of equal diameter only are illustrated in the Figures for sake of simplicity. It is clear that spots of different diameters and distributed according to any pattern or even randomly can be defined, as long as the whole volume of the target volume (Vt) is thus covered.

As shown in Figure 3(b) such spot positions pattern on a line parallel to the X-axis yields a wavy dose distribution pattern with maxima at the level of the beamlets axes, and with minima at mid-distance between two adjacent beamlet axes. The dose distribution pattern becomes more complex when the PBS scans a second series of spots parallel to the axis (x) and offset with respect to the beamlets (B1, B2) represented in Figure 3(b), depending on whether the beamlets in the second line are aligned or staggered with respect to the beamlets of the first line. This shows how important it is for the skilled person to define the best spot positions pattern (x, y) as possible. This is within the competence of the skilled person.

Figure 2(b) shows a rectangular array of spots on which beamlets are successively centred. Three distances between adjacent spots are defined: the distance L14 between beamlets B1 and B4 aligned along the Y-axis, the distance L12 between beamlets B1 and B2 aligned along the X-axis, and the distance L15 between beamlets B1 and B5 aligned along a diagonal of the rectangular array, with L14 < L12 < L15. Figures 2(c) to 2(e) illustrate the resulting cumulated dose deposited onto a specific volume (vi) centred at location B1 by two adjacent spots isolated from the other spots, depending on the distance (L12, L14, L15) separating them. For example, referring to Figure 2(b), the specific volume (vi) is centred on position B1, at spot S1. It can be seen in Figure 2(c), wherein the adjacent spots aligned along the Y-axis are the closest, separated by a distance L14, the Gaussian of beamlet B1 centred on spot S1 delivers its maximum dose at the position of the corresponding specific volume(vi) centred on the spot S1, while the Gaussian of beamlet B4 centred on spot S4 delivers a lower, albeit significant dose to the specific volume (vi) corresponding to spot S1. A significant dose is a dose defined as Dj ≥ Dmin, wherein Dmin is a lower dose boundary. A dose below Dmin does not significantly increase the total dose deposited in the spot. The dose, Dmin, could be a percentage of the total dose (Σ_{{tⱼ}} Dj) to be deposited onto a given specific volume or a percentage of an average of doses to be deposited onto the critical volume (Vc). For example, Dmin could be comprised between 1 and 10%, preferably between 2 and 5% of the total dose (Σ_{{tⱼ}} Dj) to be deposited onto a specific volume, or of an average of the doses to be deposited on the spots of the critical volume (Vt). Alternatively, Dmin can be an absolute value, whose value depends on the tissue being irradiated, e.g., taking account of NTCP thereof.

Similarly, Figure 2(d) shows the Gaussians of beamlets (B1, B2) centred on spots S1, S2, respectively. Spots S1, S2 are aligned along the X-axis and are separated by a distance L12 > L14. The beamlet B2 deposits at the specific volume centred on spot S1 a lower dose than the beamlet B4 illustrated in Figure 2(c), but that is still substantially higher than Dmin. Finally, Figure 2(e) shows that, in that example, the dose deposited by beamlet B5) centred on spot S5 deposits an unsignificant dose (i.e., Dj > Dmin) onto the specific volume centred on spot B1. Spot S5 is aligned along a diagonal of the rectangular array and is separated from spot S1 by a distance L15 (cf. Figure 2(b)). Determining the critical distance separating first and second beamlets / spots such that the first beamlet centred on a first spot deposits an unsignificant dose Dj < Dmin onto the specific volume (vi) centred on the adjacent second spot is important in the computing of the cumulative dose deposition time, ΔTi = max({tj}) - min({tj}), as the dose deposition contribution onto a given specific volume by any beamlet separated from the spot corresponding to the given specific volume by more than the critical distance is not considered for the determination of the cumulative dose deposition time, ΔTi. For example, in the case illustrated in Figures 2(a) to 2(e), the cumulative dose deposition time, ΔTi onto a specific volume (vi) is computed taking into account the beamlets centred on adjacent spots aligned along the X-axis and the Y-axis only, and not along the diagonal of the rectangular array, as the dose contribution in the latter case is not relevant (i.e., < Dmin).

The lower dose boundary (Dmin) can be defined as an absolute dose value, below which a dose is considered to have an insignificant effect. Alternatively, the lower dose boundary (Dmin) can be defined as a percentage of the total dose deposited on the specific volume (vi). For example, Dmin can be at least 5% of Σ_{{tⱼ}} Dj deposited onto the specific volume (vi), preferably at least 10% of Σ_{{tⱼ}} Dj.

### PSEUDO-MEDIATRIX (M)

When the scarf sequence unit cell defined in EP3932482 defines *"straight scarves"* only, the present invention proposes to define *"folded scarves"* matching the geometries of the tumours. To achieve this goal, the method of the present invention comprises defining a pseudo-mediatrix (M) characterizing a geometry of the target perimeter (Pt), An example of pseudo-mediatrix (M) is illustrated in Figure 2(a), (cf. double line). The pseudo-mediatrix (M) defines a backbone of the target area (At), which is followed by the irradiation path (IP) defined by the sequence of spots (Sj), the beamlets sequentially aim at. Several methods can be applied to define the pseudo-mediatrix (M). For example, the pseudo-mediatrix can be determined by any one of the best fit ellipse method, the mid-point method, or the geometric median method, described in continuation. Other methods can be used to define the pseudo-mediatrix (M) as long as it defines a backbone of the geometry of the target area (At).

### Best fit ellipse method

The best fit ellipse method is illustrated in Figures 6(a) & 6(b) and in Figures 7(a) & 7(b). A set of reference points (RP) is first defined, which are distributed over a whole of the target area (At), as shown in Figure 6(a). Since an array of spots (Sj) is already defined, it is preferred that the reference points (RF) are centres of the spots (Sj), as shown in Figure 7(a). A first reference point (RP) / spot (Sj) is selected, and a radius of influence (R) is defined. A set influence points is determined defined as the reference points (RP) / spots (Bj) enclosed within a circle of influence of radius of influence (R) centred on the first reference point (RP) / spot (Bj).

An ellipse of influence is then computed, having a same second moment of inertia as the set of all influence points in the circle of influence. The major axis of the ellipse of influence is identified and a flow vector is defined starting from the first reference point (RP) / spot (Bj), of direction parallel to the major axis, and of arbitrary length,

The foregoing steps are repeated for all reference points / spots with circles of influence of the same radius of influence (R), and with flow vectors of same arbitrary length. The flow vectors can be connected to one another to define flow lines, as shown in Figures 6(b) and 7(b). The pseudo-mediatrix can be defined by selecting one flow line extending from one end to another end of the target perimeter (Pt) as forming the pseudo-mediatrix (M).

As can be seen by comparing Figure6(a) and 6(b), any reference point / spot positioned at one end of the target perimeter can be selected regardless of whether it is centred or adjacent to an outer boundary of the target perimeter (Pt). To save computing time, the processor carrying out the foregoing operations can select as the first reference point (RP) / spot (Bj) a reference point / spot located at one end of the target perimeter. Once the flow vector is defined for the first time, repeating the process with a second reference point (RP) / spot (Bj) which is best aligned with the flow vector and is closest to the first reference point / spot.

### Mid-point Method

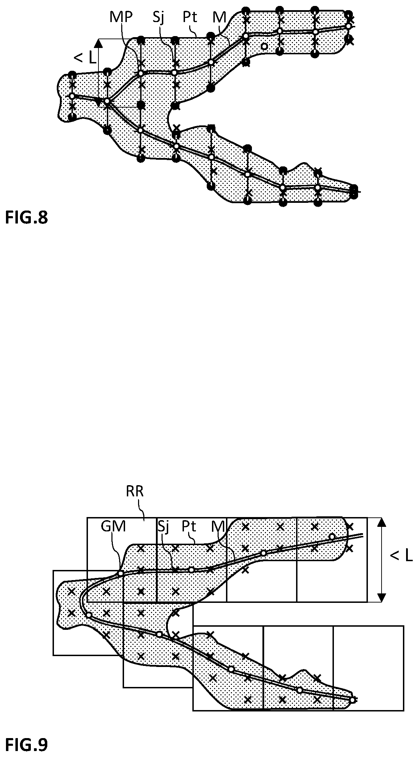

An alternative method for defining the pseudo-mediatrix (M) is the mid-point method illustrated in Figure 8. In the mid-point method, a first connecting segment is defined, having a first direction and intersecting the target perimeter at two intersecting points. A segment length is defined as a distance separating the two intersecting points. The mid-point of the first connecting segment is defined as the point located at a centre of the first connecting segment. The foregoing steps are repeated with further connecting segments parallel to the first direction. The pseudo-mediatrix can be formed by connecting all mid-points to define the pseudo-mediatrix (M). It is preferred that the first connecting segment and further connecting segments each passes through the centre of at least one spot (Sj), preferably of at least two spots (Sj).

### Geometric median method

Yet an alternative method for defining the pseudo-mediatrix (M) is the geometric median method illustrated in Figure 9. The geometric median method starts by the definition of a reference rectangle (RR) of given dimensions and orientation. The target area (At) is then paved with a series of reference rectangles (RR) such as to cover a whole of the target area. The reference rectangles (RR) may or may not overlap. The spots (Sj) enclosed in each reference rectangle (RR) are identified and the geometric median of the spots (Sj) enclosed in each reference rectangle (RR) is determined. The geometric median thus determined for each reference rectangle (RR) forms a corresponding dot of the pseudo-mediatrix (M), In case two reference rectangles (RR) overlap and both enclose a same spot (Sj) the same spot (Sj) is accounted twice, once for each reference rectangle (RR), for determining the geometric median associated to each reference rectangle (RR). The pseudo-mediatrix (M) is defined by connecting the dots thus formed to form the pseudo-mediatrix (M).

The geometric median of a set of spots enclosed in a reference rectangle (RR) is the point minimizing the sum of distances to the spots. In a Euclidian space, the geometric median (GM) of a set of m spots (Sj) enclosed in a reference rectangle (RR) satisfies the equation, ${\sum }_{j}^{m} \left(Sj-Gm\right) / \left|Sj-GM\right|$.

### DEFINITION OF AN IRRADIATION SEQUENCE

The irradiation path (IP) defining the sequence of spots (Sj) to be sequentially irradiated by beamlets can be divided into a series of path sections which connect a number of adjacent spots distributed on one side or on either side of the pseudo-mediatrix. Each path section intersects or crosses once the pseudo-mediatrix (M) at different points distributed along a length of the pseudo-mediatrix. The path sections are connected to one another two by two forming a series of return path sections of length smaller than or equal to twice the HDR-scanning distance (2L).

### Reference boxes (RB)

Each path section can be defined by introducing a rectangular reference box (RB) having major edges of length (Lb) equal to or smaller than the scanning distance (L) and having minor edges of width (Wb < Lb) equal to or smaller than the shortest distance (ds) separating two adjacent spots along a direction parallel to the minor edges. The target area (At) can be paved with reference boxes (RB) such that all spots are enclosed in at least one reference box, preferably in a single reference box, and such that the major median of each reference box (RB) crosses the pseudo-mediatrix (M) at least once, preferably once only.

As shown in Figures 10(a) to 10(c), the reference boxes (RB) can pave the target area in different ways. For example, Figure 10(a) shows a target area (At) with a rectangular array of spots (Sj) and with a pseudo-mediatrix (M) obtained by the best-fit ellipse method as illustrated in Figure 7(a) & 7(b). The reference boxes (RB) are all aligned with the major edges parallel to the Y-axis. It can be seen that one spot (Sj) is enclosed in two different reference boxes (RB). At the time of joining the path segments, that spot is linked to the other spots enclosed in one only of the two reference boxes (RB) and is ignored upon linking the spots enclosed in the other one of the two reference boxes (RB).

Figure 10(b) shows an alternative example, wherein the same target area (At) as in Figure 10(a) comprises a hexagonal array of spots. In this example, the pseudo-mediatrix was defined by the mid-point method as illustrated in Figure 8. The reference boxes (RB) are all aligned at 60° with the X-axis, in alignment with the spots. It this embodiment, all spots are enclosed in one and one only reference box.

Figure 10(c) shows yet an alternative example, wherein the same target area (At) as in Figure 10(a) comprises the same rectangular array of spots (Sj). The pseudo-mediatrix (M) was here defined using the geometric median method as illustrated in Figure 9. The reference boxes (RB) are here aligned with their major median perpendicular to the pseudo-mediatrix (M). As in the embodiment of Figure 10(a), one spot can be enclosed in two reference boxes (RB).

Figures 10(a) to 10(c) show first that the reference boxes (RB) can pave the target area (At) according to different criteria and yield different paving patterns, but all suitable for defining an optimal irradiation sequence. Second, the method used to define the pseudo-mediatrix (M) is not critical, as long as it defines a "backbone" characteristic of the geometry of the target area (At) and guiding the positioning of the reference boxes (RB) along the pseudo-mediatrix.

### Joining the spots to define a continuous path or irradiation path (IP)

A continuous path or irradiation path (IP) passing once and once only over each spot (Sj) can now be defined, ensuring that a sum of a percentile of all the doses deposited by one or more beamlets onto the target volume are deposited at a ultra-high dose deposition rate (HDR),

The path sections are first defined in each individual reference box, by joining the spots (Sj) enclosed in one reference box (RB) to the nearest neighbouring spot in the same reference box. Because of the size of the reference boxes (RB), the maximum length of each path section is therefore close to and smaller than the scanning length (L). If a spot is enclosed in more than one reference box, that spot is considered for one reference box (RB) only and is ignored when defining the path sections in the other reference boxes (RB) enclosing that spot. Next, the path sections of each reference box (RB) are connected to one another two by two to form the continuous path. This is generally performed by joining a spot at an end point of a first path section enclosed in a first reference box (RB) to an end point of a second path section enclosed in a second reference box, adjacent to the first reference box. This can be achieved in different ways.

In one embodiment illustrated in Figures 4.1 to 4.7 and 11(a), one end point of the first path section is joined to the end point of the second path section located closest thereto. This maximizes the variations between longest and shortest cumulative dose deposition times (ΔTi, between the adjacent spots (Bj) of the first and second path sections. This is illustrated graphically in Figures 4.1 to 4.7, with,
- the shortest cumulative dose deposition times (ΔTi is at spot (S4) illustrated in Figure 4.4, with a cumulative dose deposition time (ΔTi(D4)) of only t5 - t4, whilst
- the longest cumulative dose deposition times (ΔTi) is at spots (S1) and (S7) illustrated in Figures 4.1 and 4.7, with a cumulative dose deposition time (ΔTi(D1) = ΔTi(D7)) as long as t7 - t1.

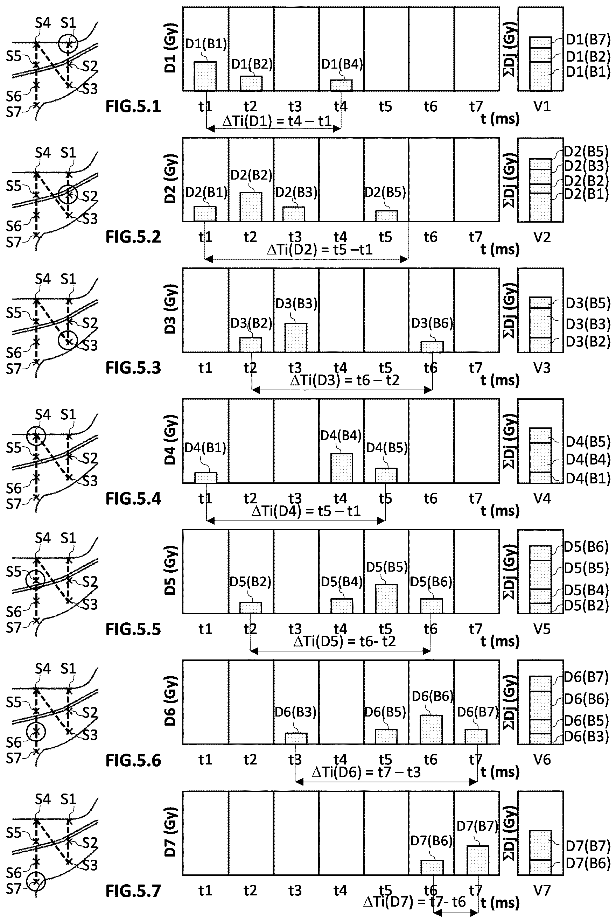

In an alternative embodiment, illustrated in Figures 5.1 to 5.7 and 11(c), one end point of the first path section is joined to the end point of the second path section located furthest away therefrom. This minimizes the variations between longest and shortest cumulative dose deposition times (ΔTi, between the adjacent spots (Bj) of the first and second path sections. This is illustrated graphically in Figures 5.1 to 5.7, with,
- the shortest cumulative dose deposition times (ΔTi at spot (S7) illustrated in Figure 5.7, with a cumulative dose deposition time (ΔTi(D7)) of only t7 - t6, whilst
- the longest cumulative dose deposition times (ΔTi) is at spots (S2) illustrated in Figure 5.2, with a cumulative dose deposition time (ΔTi(D2)) as long as t6 - t1.

### IRRADIATION SEQUENCE ACCORDING TO THE PRESENT INVENTION

Figures 4.1 to 4.7 and 5.1 to 5.7 illustrate the cumulative dose deposition times (ΔTi) at each of a subarray of spots (S1 to S7) belonging to two adjacent path sections, joined to one another in different ways, as discussed supra. The spots subarrays are illustrated on the left-hand side of each of Figures 4.1 to 4.7 and 5.1 to 5.7, and the spot concerned by the cumulative dose deposition times (ΔTi) is surrounded by a circle. The graphs in the middle section illustrate the doses (Dj) deposited at a time (tj). The time (tj) is divided into seven time-fractions (t1 to t7) required for scanning the seven spots (S1 to S7). The graphs on the right-hand side indicate the cumulative doses ( ${\sum }_{1}^{7} Dj$).

The dose deposition contribution on a given specific volume (vi- from beamlets aimed at neighbouring spots (Sj) is as illustrated in Figures 2(b) to 2(e). Beside the beamlet aligned on the spot (Sj) corresponding to the given specific volume (vi), the highest contribution comes from beamlets aimed at neighbouring spots (Sj) aligned along the Y-axis with the spot corresponding to the specific volume concerned, which are separated from one another by a distance L14. A lower contribution comes from beamlets aimed at neighbouring spots (Sj) aligned along the X-axis and separated by a distance L12. Finally, no significant contribution in the dose deposition is computed from beamlets aimed at adjacent spots aligned along a diagonal and separated from one another by a distance L35.

In Figures 4.1 to 4.7, the path segments are joined by linking end points located closest to one another (= short connection path), whilst in Figures 5.1 to 5.7, they are joined by linking end points located furthest away from one another (= long connection path). It can be seen by comparing Figures 4.1 to 4.7 with Figures 5.1 to 5.7, that the cumulative dose deposition times (ΔTi) differ depending on the implemented irradiation sequence of spots. The cumulative dose ( ${\sum }_{1}^{7} Dj$) deposited on each specific volume (vi), however, does not vary between the two irradiation sequences.

As already mentioned supra, the variation of cumulative dose deposition times (ΔTi) between different spots (Sj) is larger with the short connection path illustrated in Figures 4.1 to 4.7 than with the long connection path illustrated in Figures 5.1 to 5.7. For sake of simplifying the discussion, it is herein assumed that the time period (tk - t(k-1)) separating the ends of dose depositions by beamlets B(k-1) and Bk onto two adjacent spots Sj, S(j+1) along the connection path is equal for all pairs of adjacent spots (Sj, S(j+1)). The time period (tk - t(k-1)) is referred to as the "fraction time unit" in Figures 4.1 to 4.7 and 5.1 to 5.7. This assumption is quite reasonable as the time required for depositing a dose with a beamlet centred on a first spot is longer than the time required for scanning the beamlet to an adjacent spot, whose distances do not vary much along the connection path. The shortest cumulative dose deposition time (ΔTi) in both short and long connection paths is of 1 fraction time unit (ΔTi(D4) = t5 - t4 at spot S4 in Figure 4.4 and ΔTi(D7) = t7 - t6 at the same spot, but numbered S7 in Figure 5.7). The longest cumulative dose deposition time (ΔTi) with the short connection path is 7 fraction time units long at spot S1 (ΔTi(D1) = t7 - t1 at spot S1 in Figure 4.1), whilst the longest cumulative dose deposition time (ΔTi) with the long connection path is only 5 fraction time units long (ΔTi(D2) = t5 - t1 at spot S2 in Figure 5.2, ΔTi(D3) = t6 - t2 at spot S3 in Figure 5.3, ΔTi(D4) = t5 - t1 at spot S4 in Figure 5.4, ΔTi(D5) = t6 - t2 at spot S5 in Figure 5.5, and ΔTi(D6) = t7 - t3 at spot S6 in Figure 5.6). The same rationale applies, *mutatis mutandis,* when the delivery time is not the same for all the spots.

Table 1 lists the cumulative dose deposition time (ΔTi) in fraction time unit units at each spot (Sj) of a sequence along the short connection path illustrated in Figures 4.1 to 4.7, and of a sequence along the long connection path illustrated in Figures 5.1 to 5.7. The average and standard deviations are also indicated. The short connection path yields a longer average cumulative dose deposition time (ΔTi) of 4.86 fraction time units, with larger variations as discussed supra, with a standard deviation of 1.86 fraction time units. By contrast, the long connection path has a shorter cumulative dose deposition time (ΔTi) of 4.43 fraction time units with a narrower distribution with a standard deviation of 1.13 fraction time units. This is not to say that the long connection path is always preferred, as specific volumes (vi) containing more healthy cells must require HDR irradiation, whilst specific volumes containing tumoural cells only can afford to be irradiated at lower rates and even at CDR. The choice of one or the other options between short or long connection depends on the geometry of the target volume (Vt) and the positions of the spots which must be irradiated at HDR and those which are merely preferably irradiated at HDR.

**Table 1: cumulative dose deposition time (ΔTi) at each spot S1-S7 depending on the irradiation sequence characterized by a short connection path (cf. Figures 4.1-4.7) and a long connection path (cf. Figures 5.1-5.7) (ΔTi expressed in fraction time units)**

| | short conn. FIG.4 | long conn. FIG.5 |
|---|---|---|
| S1 | 7 | 4 |
| S2 | 6 | 5 |
| S3 | 4 | 5 |
| S4 | 2 | 5 |
| S5 | 4 | 5 |
| S6 | 4 | 5 |
| S7 | 7 | 2 |
| AVG | **4.86** | **4.43** |
| STDEV | **1.86** | **1.13** |

By enclosing the path fractions within reference boxes (RB) with major edges of length (Lb) equal to or smaller than the scanning length (L), joining two adjacent path fractions defines a trajectory wherein the cumulated doses can be deposited at HDR into each specific volume (vi) corresponding to the spots forming the trajectory.

Sensitive zones of the target area (At) are two or more spots belonging to adjacent reference boxes (RB), whose path sections are not directly joined together, as for example the pair of spots identified by the ellipse in Figure 11(a). The two spots are aligned along the Y-axis, such that a beamlet aimed at one of the two spots deposits a substantial dose on the neighbouring spot too. Their respective irradiations are, however, separated by a long period of time, too long to guarantee a dose deposition at HDR onto these two spots. In such cases, care must be taken to limit the occurrence of such non-joined adjacent pairs of spots in zones of the target volume (Vt) containing mostly tumoural cells and very little healthy cells, since killing tumoural cells is not dependent on the dose deposition rate. Dose deposition at ultra-high dose deposition rates (HDR) is beneficial for the preservation of healthy cells to an irradiation operation.

The present invention proposes a simple and reproducible method for defining an irradiation scanning sequence of spots characterizing a target area (At) of a target volume (Vt) of complex geometry, ensuring that doses (Dj) are deposited at a ultra-high dose deposition rate (HDR) onto a significant fraction of specific volumes (vi) defining the target volume (Vt)

| **REF #** | **Feature** |
|---|---|
| At | target area |
| GM | Geometric median |
| HDR | High dose rate |
| IP | Irradiation path |
| L | HDR-scanning distance = distance a particle beam can scan in an up-and-down return path while ensuring that the dose (Dj) is deposited at HDR deposition rate into all spots touched by the particle beam along the up-and-down return path thereof |
| MP | Mid-point |
| P0 | Projection plane |
| Pt | target perimeter |
| RB | Reference box |
| RP | Reference point |
| RR | Reference rectangle |
| Si | Spot number j |
| Vt | target volume |
| Δ*Tᵢ* | Cumulative dose deposition time onto a specific volume (vi) |

## Claims

1. A method for defining a scanning sequence for treatment by radiation with charged particles beams, preferably with proton beams, wherein the method ensures that doses (Dj) are deposited by pencil beam scanning (PBS) in an energy monolayer onto a target volume (Vt) comprising tumoral cells enclosed within a peripheral surface and at a ultra-high dose deposition rate (HDR) onto a significant fraction of specific volumes (vi) defining a critical volume (Vc) comprising healthy cells, wherein HDR is defined as a dose deposition rate, HDR = Σ_{{tⱼ}} Dj/ΔTi ≥ 1 Gy / s, wherein Dj is a dose deposited onto a specific volume (vi) of the (Vc) by a j^{th} pencil beam at time tj after beginning of the irradiation, and wherein ΔTi = max({tj}) - min({tj}), is a time spanning between an initial dose deposition time (min({tj})) when a first dose ≥ Dmin is deposited into the specific volume and a final dose deposition time (max({tj})) when a last dose ≥ Dmin is deposited into the specific volumewherein Dmin is a lower dose boundary, the method comprising,
• defining a target area (At) bounded by a target perimeter (Pt) by projection of the target volume onto a surface plane (P0 = (Y, Z)) normal to a central irradiation axis (X),
• defining a spot diameter and spot position pattern on the surface plane (P0) of spots (Sj) distributed at least within the target perimeter (Pt) such as to cover at least a whole area defined within the target perimeter (Pt),
• establishing a scarf scanning sequence defining a continuous path connecting all the spots (Sj) to one another and configured for minimizing the cumulative dose deposition time (Δ*Tᵢ*) in all the specific volumes (vi), such as to ensure HDR-deposition into all specific volume (vi),
**characterized in that,** the scarf scanning sequence is established as follows,
• for a given value of the charged particles beam current, defining a HDR-scanning distance (L) a particle beam can scan in a two-way return path while ensuring that the dose (Dj) is deposited at HDR deposition rate into all specific volumes (vi) touched by the particle beam along the two-way return path thereof,
• defining a pseudo-mediatrix (M) characterizing a geometry of the target perimeter (Pt), wherein the pseudo-mediatrix (M) defines a backbone of the target area (At), which is followed by an irradiation path (IP) defined by the scanning sequence of spots (Sj), the beamlets sequentially aim at.
• the continuous path comprises a series of path sections which connect a number of adjacent spots distributed on one side or on either side of the pseudo-mediatrix wherein,
∘ each path section intersects or crosses once the pseudo-mediatrix (M) at different points distributed along a length of the pseudo-mediatrix,
∘ the path sections are connected to one another two by two forming a series of return path sections of length smaller than or equal to the HDR-scanning distance (L).

2. Method according to claim 1, wherein the return path sections are formed by connecting an end spot of a first path-section defined as a last spot connected in the first path section to a second path section adjacent to the first path section by connecting the end spot of the first section to a spot of the second path-section located either,
• at a shortest distance from and on the same side of the pseudo-mediatrix (M) as the last spot, or
• at a longest distance from and on the other side of the pseudo-mediatrix (M) as the last spot.

3. Method according to claim 1 or 2, wherein the pseudo-mediatrix (M) is defined as follows,
• define a set of reference points (RP) distributed over a whole of the target area (At),
• select a first reference point (RP),
• define a radius of influence (R),
• define a set of influence points defined as the reference points (RP) enclosed within a circle of influence of radius of influence (R) centred on the first reference point (RP)
• compute an ellipse of influence having a same second moment of inertia as the set of all influence points in the circle of influence,
• identify the major axis of the ellipse of influence,
• define a flow vector starting from the first reference point (RP), of direction parallel to the major axis, and of arbitrary length,
• repeat the foregoing steps for all reference points (RP) with circles of influence of the same radius of influence (R),
• connect the flow vectors to one another to define flow lines,
• select one flow line extending from one end to another end of the target perimeter (Pt) as forming the pseudo-mediatrix (M).

4. Method according to the preceding claim 3, wherein the reference points (RP) are centres of spots (Sj).

5. Method according to claim 1 or 2, wherein the pseudo-mediatrix (M) is defined as follows,
• Defining a first connecting segment of first direction intersecting the target perimeter at two intersecting points, wherein a segment length is defined as a distance separating the two intersecting points,
• Defining a first mid-point (MP) of the first connecting segment, defined as the point located at a centre of the connecting segment,
• Repeating the foregoing steps with further connecting segments parallel to the first direction,
• Connecting all mid-points (MP) to define the pseudo-mediatrix (M).

6. Method according to the preceding claim 5, wherein the first connecting segment and further connecting segments each passes through the centre of at least one spot (Sj), preferably of at least two spots (Sj).

7. Method according to claim 1 or 2, wherein the pseudo mediatrix (M) is defined as follows,
• Define a reference rectangle of given dimensions and orientation,
• Pave the target area (At) with a series of reference rectangles such as to cover a whole of the target area,
• For a first reference rectangle, identify the spots (Sj) enclosed therein,
• Determine the geometric median (GM) of the spots (Sj) enclosed in the first reference rectangle, wherein the geometric median forms a first dot of the pseudo-mediatrix (M),
• Repeat the last two steps for all the reference rectangles to form a string of dots,
• Connecting the dots thus formed to form the pseudo-mediatrix (M).

8. Method according to any one of the preceding claims, wherein the scarf scanning sequence is established as follows,
• Define a rectangular reference box (RB) with major edges of length (Lb) and with minor edges of width (Wb < Lb) having a major median extending parallel to the major edges, wherein Lb < L and Wb is smaller than twice a shortest distance (ds) separating the centres of two adjacent spots (Sj) (i.e., Wb < 2 ds),
• Pave the target area (At) with a series of reference boxes (RB), with the major median crossing the pseudo-mediatrix (M) and forming therewith a given angle, preferably of 90°, such as to enclose all the spots (Sj) in at least one reference box,
• Defining a path section with spots (Sj) enclosed in one reference box, each spot (Sj) belonging to a single path section even if enclosed in more than one reference box,
• Connecting the path sections to one another two by two to form the continuous path.

9. Method according to any one of the preceding claims, wherein the lower dose boundary (Dmin) is defined either
• as an absolute dose value, below which a dose is considered to have an insignificant effect, or
• as a percentage of the total dose deposited to the specific volume (vi), preferably Dmin is at least 5% of Σ_{{tⱼ}} Dj deposited onto the specific volume (vi), more preferably at least 10% of Σ_{{tⱼ}} Dj.

## Patentansprüche

1. Verfahren zum Definieren einer Scansequenz zur Behandlung durch Strahlung mit geladenen Teilchen, vorzugsweise mit Protonenstrahlen, wobei das Verfahren sicherstellt, dass Dosen (Dj) mittels Pencil-Beam-Scanning (PBS) in einer Energiemonoschicht auf ein Zielvolumen (Vt), das Tumorzellen umfasst, die in einer peripheren Oberfläche eingeschlossen sind, und mit einer ultrahohen Dosisdepositionsrate (HDR) auf einen signifikanten Anteil spezifischer Volumina (vi), die ein kritisches Volumen (Vc) definieren, das gesunde Zellen umfasst, deponiert werden, wobei HDR als Dosisdepositionsrate, HDR = Σ_{{tⱼ}}Dj/ΔTi ≥ 1 Gy/s, definiert ist, wobei Dj eine Dosis ist, die durch einen j^{-te} Pencil-Beam zum Zeitpunkt tj nach Bestrahlungsbeginn auf ein spezifisches Volumen (vi) des (Vc) deponiert wird, und wobei ΔTi = max({tj}) - min({tj}) eine Zeitspanne zwischen dem Zeitpunkt der ersten Dosisdeposition (min({tj}))), an dem eine erste Dosis D ≥ Dmin in das spezifische Volumen deponiert wird, und einem Zeitpunkt der letzten Dosisdeposition (max({tj})) ist, an dem eine letzte Dosis ≥ Dmin in das spezifische Volumen deponiert wird, wobei Dmin eine untere Dosisgrenze ist, wobei das Verfahren umfasst
- Definieren einer Zielfläche (At), die durch einen Zielumfang (Pt) begrenzt wird, durch Projektion des Zielvolumens auf eine Oberflächenebene (P0 = (Y, Z)), normal zu einer zentralen Bestrahlungsachse (X),
- Definieren eines Punktdurchmessers und eines Punktpositionsmusters auf der Oberflächenebene (P0) von Punkten (Sj), die mindestens innerhalb des Zielumfangs (Pt) verteilt sind, sodass sie mindestens eine gesamte innerhalb des Zielumfangs (Pt) definierte Fläche abdecken,
- Erstellen einer Scarf-Scansequenz, die einen kontinuierlichen Pfad definiert, der alle Punkte (Sj) miteinander verbindet und so konfiguriert ist, dass die kumulative Dosisdepositionszeit (Δ*Tᵢ*) in allen spezifischen Volumina (vi) minimiert wird, um die HDR-Deposition in alle spezifischen Volumina (vi) sicherzustellen,
**dadurch gekennzeichnet, dass** die Scarf-Scansequenz wie folgt erstellt wird
- für einen gegebenen Wert des Strahlstroms geladener Teilchen, Definieren einer HDR-Scandistanz (L), die ein Teilchenstrahl in einem bidirektionalen Rückweg scannen kann, wobei sichergestellt wird, dass die Dosis (Dj) mit der HDR-Depositionsrate in alle spezifische Volumina (vi) deponiert wird, die vom Teilchenstrahl entlang des bidirektionalen Rückwegs berührt werden,
- Definieren einer Pseudo-Mediatrix (M), die eine Geometrie des Zielumfangs (Pt) charakterisiert, wobei die Pseudo-Mediatrix (M) ein Rückgrat der Zielfläche (At) definiert, dem ein Bestrahlungspfad (IP) folgt, der durch die Scansequenz von Punkten (Sj) definiert ist, auf die die Strahlenbündelchen nacheinander zielen,
- der kontinuierliche Pfad eine Reihe von Pfadabschnitten umfasst, die eine Anzahl benachbarter Punkte verbinden, die auf einer Seite oder auf beiden Seiten der Pseudo-Mediatrix verteilt sind, wobei
- jeder Pfadabschnitt die Pseudo-Mediatrix (M) genau einmal an verschiedenen Punkten entlang ihrer Länge schneidet oder kreuzt,
- die Pfadabschnitte paarweise miteinander verbunden sind, indem sie eine Reihe von Rückwegabschnitten bilden, deren Länge kleiner oder gleich der HDR-Scandistanz (L) ist.

2. Verfahren nach Anspruch 1, wobei die Rückwegabschnitte gebildet werden, indem ein Endpunkt eines ersten Wegabschnitts, der als letzter verbundener Punkt im ersten Wegabschnitt definiert ist, mit einem zweiten, an den ersten Wegabschnitt angrenzenden Wegabschnitt verbunden wird, indem der Endpunkt des ersten Abschnitts mit einem Punkt des zweiten Wegabschnitts verbunden wird, der sich entweder
- in kürzestem Abstand von und auf derselben Seite der Pseudo-Mediatrix (M) wie der letzte Punkt befindet oder
- in größtem Abstand von und auf der anderen Seite der Pseudo-Mediatrix (M) als letzter Punkt befindet.

3. Verfahren nach Anspruch 1 oder 2, wobei die Pseudo-Mediatrix (M) wie folgt definiert wird:
- Definieren einer Menge von Referenzpunkten (RP), die über die gesamte der Zielfläche (At) verteilt sind,
- Auswählen eines ersten Referenzpunkts (RP),
- Definieren eines Einflussradius (R),
- Definieren eines Satzes von Einflusspunkten, die als die Referenzpunkte (RP) definiert sind, die innerhalb eines Einflusskreises mit Einflussradius (R), zentriert auf den ersten Referenzpunkt (RP) eingeschlossen sind,
- Berechnen einer Einflussellipse, die ein selbes zweites Trägheitsmoment besitzt wie der Satz aller Einflusspunkte im Einflusskreis,
- Identifizieren der Hauptachse der Einflussellipse,
- Defnieren eines Flussvektors, der vom ersten Referenzpunkt (RP) ausgeht, dessen Richtung parallel zur Hauptachse verläuft und der beliebige Länge hat,
- Wiederholen der vorangehenden Schritte für alle Referenzpunkte (RP) mit Einflusskreisen gleichen Einflussradius (R),
- Verbinden der Flussvektoren, um Flusslinien zu definieren,
- Auswählen einer Flusslinie, die sich von einem Ende zum anderen Ende des Zielumfangs (Pt) erstreckt und die Pseudo-Mediatrix (M) bildet.

4. Verfahren nach dem vorstehenden Anspruch 3, wobei die Referenzpunkte (RP) Mittelpunkte von Punkten (Sj) sind.

5. Verfahren nach Anspruch 1 oder 2, wobei die Pseudo-Mediatrix (M) wie folgt definiert wird:
- Definieren eines ersten Verbindungssegments erster Richtung, das den Zielumfang in zwei Schnittpunkten schneidet, wobei eine Segmentlänge als Abstand zwischen den beiden Schnittpunkten definiert ist,
- Definieren eines ersten Mittelpunkts (MP) des ersten Verbindungssegments, definiert als der Punkt, der sich in einer Mitte des Verbindungssegments befindet,
- Wiederholen der vorangehenden Schritte mit weiteren Verbindungssegmenten parallel zur ersten Richtung,
- Verbinden aller Mittelpunkte (MP), um die Pseudo-Mediatrix (M) zu definieren.

6. Verfahren nach dem vorstehenden Anspruch 5, wobei das erste Verbindungssegment und weitere Verbindungssegmente jeweils durch den Mittelpunkt mindestens eines Punkts (Sj), vorzugsweise mindestens zweier Punkte (Sj), verlaufen.

7. Verfahren nach Anspruch 1 oder 2, wobei die Pseudo-Mediatrix (M) wie folgt definiert wird:
- Definieren eines Referenzrechtecks mit gegebenen Abmessungen und gegebener Ausrichtung,
- Belegen der Zielfläche (At) mit einer Reihe von Referenzrechtecken, sodass eine gesamte Zielfläche abgedeckt wird,
- Identifizieren für ein erstes Referenzrechteck der darin eingeschlossenen Punkte (Sj),
- Bestimmen des geometrischen Medians (GM) der Punkte (Sj), die im ersten Referenzrechteck eingeschlossen sind, wobei der geometrische Median einen ersten Punkt der Pseudo-Mediatrix (M) bildet,
- Wiederholen der letzten zwei Schritte für alle Referenzrechtecke, um eine Punktkette zu bilden,
- Verbinden der so gebildeten Punkte, um die Pseudo-Mediatrix (M) zu bilden.

8. Verfahren nach einem der vorstehenden Ansprüche, wobei die Scarf-Scansequenz wie folgt erstellt wird:
- Definieren eines rechteckigen Referenzrahmens (RB) mit Hauptkanten einer Länge (Lb) und Nebenkanten einer Breite (Wb < Lb) mit einem Hauptmedian, der sich parallel zu den Hauptkanten erstreckt, wobei Lb < L und Wb kleiner als das Doppelte eines kürzesten Abstands (ds) ist, der die Mittelpunkte zweier benachbarter Punkte (Sj) trennt (d. h. Wb < 2 ds),
- Belegen der Zielfläche (At) mit einer Reihe von Referenzrahmen (RB), wobei der Hauptmedian die Pseudo-Mediatrix (M) kreuzt und mit dieser einen bestimmten Winkel, vorzugsweise 90°, bildet, sodass alle Punkte (Sj) in mindestens einem Referenzrahmen eingeschlossen sind,
- Definieren eines Pfadabschnitts mit Punkten (Sj), die in einem Referenzrahmen eingeschlossen sind, wobei jeder Punkt (Sj) zu einem einzigen Pfadabschnitt gehört, auch wenn er in mehr als einem Referenzrahmen eingeschlossen ist,
- paarweises Verbinden der einzelnen Pfadabschnitte, um einen durchgehenden Pfad zu bilden.

9. Verfahren nach einem der vorstehenden Ansprüche, wobei die untere Dosisgrenze (Dmin) entweder:
- als absoluter Dosiswert, unterhalb dessen eine Dosis angesehen wird, eine insignifikante Wirkung zu haben, oder
- als Prozentsatz der Gesamtdosis, die in das spezifische Volumen (vi) deponiert wird, definiert ist, wobei Dmin vorzugsweise mindestens 5% von Σ_{{tⱼ}}DJ beträgt, das auf dem spezifischen Volumen (vi) deponiert wird, bevorzugter mindestens 10% von Σ_{{tⱼ}} DJ.

## Revendications

1. Procédé de définition d'une séquence de balayage pour un traitement par irradiation avec des faisceaux de particules chargées, de préférence avec des faisceaux de protons, dans lequel le procédé garantit le dépôt de doses (Dj) par balayage à faisceau-crayon (Pencil Beams Scanning - PBS) dans une monocouche énergétique sur un volume cible (Vt) comprenant des cellules tumorales contenues dans une surface périphérique et à un débit de dépôt de dose ultra-élevé (High Dose Deposition Rate - HDR) sur une fraction significative de volumes spécifiques (vi) définissant un volume critique (Vc) comprenant des cellules saines, dans lequel le HDR est défini comme un débit de dépôt de dose, HDR = Σ_{{tⱼ}}Dj/ΔTi ≥ 1 Gy / s, dans lequel Dj est une dose déposée sur un volume spécifique (vi) du (Vc) par un j^{ème} faisceau-crayon à l'instant tj après le début de l'irradiation, et dans lequel ΔTi = max({tj}) - min({tj}) est un intervalle de temps entre un temps de dépôt de dose initial (min({tj}) lorsqu'une première dose ≥ Dmin est déposée dans le volume spécifique et un temps de dépôt de dose final (max({tj})) lorsqu'une dernière dose ≥ Dmin est déposée dans le volume spécifique, dans lequel Dmin est une limite inférieure de dose, le procédé comprenant,
- la définition d'une zone cible (At) délimitée par un périmètre cible (Pt) par projection du volume cible sur un plan de surface (P0) = (Y, Z) perpendiculaire à un axe d'irradiation central (X),
- la définition d'un diamètre de spot et d'un motif de position de spot sur le plan de surface (P0) de spots (Sj) répartis au moins à l'intérieur du périmètre cible (Pt), de manière à couvrir au moins toute la zone définie à l'intérieur du périmètre cible (Pt),
- l'établissement d'une séquence de balayage en écharpe définissant un chemin continu reliant tous les spots (Sj) entre eux et configurée pour minimiser le temps de dépôt de dose cumulé (Δ*Tᵢ*) dans tous les volumes spécifiques (vi), de manière à assurer le dépôt à HDR dans tous les volumes spécifiques (vi),
**caractérisé en ce que** la séquence de balayage en écharpe est établie comme suit,
- pour une valeur donnée du courant du faisceau de particules chargées, définir une distance de balayage HDR (L) qu'un faisceau de particules peut balayer dans un trajet d'aller-retour bidirectionnel tout en assurant que la dose (Dj) est déposée au débit de dépôt HDR dans tous les volumes spécifiques (vi) touchés par le faisceau de particules le long de son trajet de retour bidirectionnel,
- définir une pseudo-médiatrice (M) caractérisant une géométrie du périmètre cible (Pt), dans lequel la pseudo-médiatrice (M) définit un axe principal de la zone cible (At) qui est suivie par un chemin d'irradiation (IP) défini par la séquence de balayage de spots (Sj), vers lesquels les faisceaux se dirigent séquentiellement.
- le chemin continu comprenant une série de sections de chemin qui relient un certain nombre de spots adjacents répartis d'un côté ou de l'autre de la pseudo-médiatrice, dans lequel
- chaque section de chemin coupe ou croise une fois la pseudo-médiatrice (M) en différents points répartis le long d'une longueur de la pseudo-médiatrice,
- les sections de chemin sont reliées les unes aux autres deux par deux, formant une série de sections de chemin de retour de longueur inférieure ou égale à la distance de balayage HDR (L).

2. Procédé selon la revendication 1, dans lequel les sections de chemin de retour sont formées en reliant un spot d'extrémité d'une première section de chemin défini comme le dernier spot relié dans la première section de chemin à une deuxième section de chemin adjacente à la première section de chemin en reliant le spot d'extrémité de la première section à un spot de la deuxième section de chemin situé soit,
- à la distance la plus courte depuis et du même côté de la pseudo-médiatrice (M) par rapport au dernier spot, ou
- à la distance la plus longue depuis et de l'autre côté de la pseudo-médiatrice (M) par rapport au dernier spot.

3. Procédé selon la revendication 1 ou 2, dans lequel la pseudo-médiatrice (M) est définie comme suit,
- définir un ensemble de points de référence (RP) répartis sur l'ensemble de la zone cible (At),
- sélectionner un premier point de référence (PR),
- définir un rayon d'influence (R),
- définir un ensemble de points d'influence définis comme les points de référence (RP) inclus dans un cercle d'influence de rayon d'influence (R) centré sur le premier point de référence (RP)
- calculer une ellipse d'influence présentant le même deuxième moment d'inertie que l'ensemble de tous les points d'influence dans le cercle d'influence,
- identifier le grand axe de l'ellipse d'influence,
- définir un vecteur de flux partant du premier point de référence (RP), de direction parallèle à l'axe principal et de longueur arbitraire,
- répéter les étapes précédentes pour tous les points de référence (RP) avec des cercles d'influence de même rayon d'influence (R),
- relier les vecteurs de flux entre eux pour définir des lignes de flux,
- sélectionner une ligne de flux s'étendant d'une extrémité à l'autre du périmètre cible (Pt) comme formant la pseudo-médiatrice (M).

4. Procédé selon la revendication 3 précédente, dans lequel les points de référence (RP) sont des centres de spots (Sj).

5. Procédé selon la revendication 1 ou 2, dans lequel la pseudo-médiatrice (M) est définie comme suit,
- définir un premier segment de liaison de première direction coupant le périmètre cible au niveau de deux points d'intersection, dans lequel la longueur d'un segment est définie comme la distance séparant les deux points d'intersection,
- définir un premier point médian (MP) du premier segment de liaison, défini comme le point situé au centre du segment de liaison,
- répéter les étapes précédentes avec d'autres segments de liaison parallèles à la première direction,
- relier tous les points médians (MP) pour définir la pseudo-médiatrice (M).

6. Procédé selon la revendication 5 précédente, dans lequel le premier segment de liaison et d'autres segments de liaison passent chacun par le centre d'au moins un spot (Sj), de préférence d'au moins deux spots (Sj).

7. Procédé selon la revendication 1 ou 2, dans lequel la pseudo-médiatrice (M) est définie comme suit,
- définir un rectangle de référence de dimensions et d'orientation données,
- paver la zone cible (At) avec une série de rectangles de référence de manière à couvrir toute la zone cible,
- pour un premier rectangle de référence, identifier les spots (Sj) qui y sont contenus,
- déterminer la médiane géométrique (GM) des spots (Sj) contenus dans le premier rectangle de référence, dans lequel la médiane géométrique forme un premier point de la pseudo-médiatrice (M),
- répéter les deux dernières étapes pour tous les rectangles de référence afin de former une chaîne de points,
- relier les points ainsi formés pour former la pseudo-médiatrice (M).

8. Procédé selon l'une quelconque des revendications précédentes, dans lequel la séquence de balayage en écharpe est établie comme suit :
- définir une case de rectangle de référence (RB) dont les grands bords de longueur (Lb) et les petits bords de largeur (Wb < Lb) présentant une médiane principale s'étendant parallèlement aux grands bords, dans lequel Lb < L et Wb est inférieur à deux fois la plus courte distance (ds) séparant les centres de deux spots adjacents (Sj) (c.-à-d. Wb < 2 ds),
- paver la zone cible (At) avec une série de cases de référence (RB), la médiane principale traversant la pseudo-médiatrice (M) et formant avec elle un angle donné, de préférence de 90°, de manière à contenir tous les spots (Sj) dans au moins une case de référence,
- définir une section de chemin avec des spots (Sj) contenus dans une case de référence, chaque spot (Sj) appartenant à une seule section de chemin même s'il est contenu dans plus d'une case de référence,
- relier les sections de chemin les unes aux autres deux par deux pour former le chemin continu.

9. Procédé selon l'une quelconque des revendications précédentes, dans lequel la limite inférieure de dose (Dmin) est définie soit
- comme une valeur de dose absolue en dessous de laquelle une dose est considérée comme présentant un effet insignifiant, ou
- comme un pourcentage de la dose totale déposée dans le volume spécifique (vi), de préférence la Dmin est au moins égale à 5 % de Σ_{{tⱼ}} Dj déposé sur le volume spécifique (vi), plus préférentiellement au moins égale à 10 % de ∑_{{tⱼ}} Dj.
